(19) Europäisches Patentamt
European Patent Office
Office européen des brevets

(11) EP 3 031 450 A1

(12) # EUROPEAN PATENT APPLICATION

(43) Date of publication:
**15.06.2016 Bulletin 2016/24**

(21) Application number: **15199505.7**

(22) Date of filing: **11.12.2015**

(51) Int Cl.:
***A61K 9/16*** *(2006.01)* ***A61K 31/545*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **12.12.2014 TR 201415054**

(71) Applicant: **Sanovel Ilac Sanayi ve Ticaret A.S.**
**34460 Istanbul (TR)**

(72) Inventors:
- **TÜRKYILMAZ, Ali**
  **34460 Istanbul (TR)**
- **YILDIRIM, Ediz**
  **34460 Istanbul (TR)**
- **TOK, Gülcin**
  **34460 Istanbul (TR)**

(74) Representative: **Sevinç, Erkan**
**Istanbul Patent A.S.**
**Plaza-33, Büyükdere Cad. No: 33/16**
**Sisli**
**34381 Istanbul (TR)**

(54) **CEFTIBUTEN CAPSULE COMPOSITIONS**

(57) This invention is related to a novel pharmaceutical capsule composition comprising ceftibuten dihydrate having a particle size ($d_{90}$) between 10.0 μm and 20.0 μm and having a particle size ($d_{50}$) between 5.0 μm and 10.0 μm and at least one pharmaceutically acceptable excipient and its preparation.

EP 3 031 450 A1

## Description

### Field of the Invention

This invention is related to a novel pharmaceutical capsule composition comprising ceftibuten dihydrate having a particle size ($d_{90}$) between 10.0 μm and 20.0 μm and having a particle size ($d_{50}$) between 5.0 μm and 10.0 μm and at least one pharmaceutically acceptable excipient and its preparation.

### Background of the Invention

Ceftibuten dihydrate is a semisynthetic cephalosporin antibiotic for oral administration. The chemical name of Ceftibuten dihydrate is (+)-(6R,7R)-7-[(Z)-2-(2-Amino-4-thiazolyl)4-carboxycrotonamido]-8-oxo-5-thia-1-azabicyclo[4.2.0]oct-2-ene-2-carboxylic acid, dehydrate and it has the following structural formula shown in below:

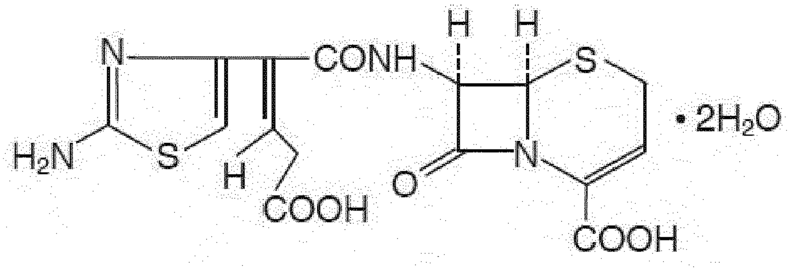

Formula

Ceftibuten dihydrate was first disclosed in US4634697. Also it is disclosed in US 4933443 and US 4812561 that ceftibuten is stable in dihydrate and trihydrate forms. Furtermore, it is also disclosed in said patents that dihydrate and trihydrate forms of ceftibuten can be filled into hard gelatin capsules for oral administration.

However, low solubility of the molecule ceftibuten in aqueous solutions due to its hygroscopic nature leads to dispersibility and solubility problems during use of solid dosage forms of formulations comprising ceftibuten. In the case that ceftibuten formulations are formulated in hard gelatin capsule form, low solubility of the capsules in gastro-intestinal liquid in body and difficulties of dissolution of ceftibuten decreases absorption of the medicine and leads to loss of effectiveness of the treatment.

Moreover, due to low water solubility of ceftibuten and its pneumatic character when it is in powder form makes it difficult to fill in other types of capsule forms.

Therefore, there is still need for new approaches in order to develop ceftibuten formulations produced in capsule form which can disperse easily and fast in body, have high absorption and bioavailability during oral use and which can easily processed during the filling step into capsule dosage forms and which is stable during the shelf-life.

### Detailed Description of the Invention

The present invention provides a novel pharmaceutical capsule composition comprising ceftibuten dihydrate having a particle size ($d_{90}$) between 10.0 μm and 20.0 μm and having a particle size ($d_{50}$) between 5.0 μm and 10.0 μm at least one pharmaceutically acceptable excipient which overcomes the above described problems in prior art and have additive advantages over them.

The main object of the present invention is to obtain a high bioavailability with improved solubility in gastro-intestinal liquid in body. The inventors have found that capsule composition comprising ceftibuten dihydrate present optimum dispersibility and solubility in the case that having a particle size ($d_{90}$) between 10 μm and 20.0 μm and having a particle size ($d_{50}$) between 5.0 μm and 10.0 μm.

In a preffered embodiment the particle size ($d_{90}$) is between 12.0 μm and 18.0 μm and the particle size ($d_{50}$) is between 5.0 μm and 8.0 μm.

One of the preffered embodiments of this invention is directed to narrow particle size distrubition of ceftibuten dihydrate composition wherein ceftibuten dihydrate has a particle size ratio between $d_{50}$ and $d_{90}$ is between 0.30 and 0.50. Preferably, the ratio between the $d_{50}$ and d $d_{90}$ is between 0.35 and 0.45.

This also helps to improve the dissolution profile and to obtain good bioavailability without causing the disadvantages of having nonuniform particle size especially when the particle size is greater than 25 or 50 micron.

As used here in, "particle size" is defined by the cumulative volume size as tested by a conventionally accepted

method which is the laser diffraction method determined by the equipment of Malvern Mastersizer 2000. "$d_{50}$" is defined by the size at which 50% by volume of the particles are finer and "$d_{90}$" is defined by the size at which %90 by volume of the particles are finer. "narrow particle size distribution" is defined by the ratio between the median particle size ($d_{50}$) and the particle size at $d_{90}$.

Another object of this invention is to provide an improved process for preparing the ceftibuten dihydrate. The inventors have found that when the final powder mixture is compacted by a compactor pressure between 25 bar and 45 bar, it is easy to fill in capsules because due to its pneumatic characteristic it is very difficult to keep it in small sizes. Furthermore when the compactor pressure is lower than 25 bar, the final powder mixure can't be made into granule form and when the compactor pressure is higher than 45 bar it is observed that the final mixture has unwanted colour changes. This is probably caused by the increase of the amount of impurities. Moreover, it is observed that the final product which is compacted by more than 45 bar has a lower dissolution profile. Thus the optiumum results are obtained when the compactor pressure is between 25 bar and 45 bar.

In a preffered embodiment the compactor pressure is between 30 bar and 40 bar.

Another object of this invention is to provide a stable capsule composition throughout the shelflife, by the help of the advantages disclosed above. When the final powder is compacted with a pressure above 45 bar, the dissolution gets worse and the powder has unwanted colour changes.

As a further embodiment, the pharmaceutical capsule composition of ceftibuten dihydrate comprise at least one pharmaceutically acceptable excipient. Suitable pharmaceutically acceptable excipients comprise but are not limited to binders, disintegrants, lubricants and glidants.

Suitable binders may comprise but not limited to microcrystalline cellulose, mannitol, spray-dried mannitol, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof. Preferably the binder is microcrystalline cellulose.

Suitable disintegrants may comprise but not limited to sodium starch glycolate, cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, polyacryline potassium, sodium alginate, corn starch, alginic acid, alginates, ion-exchange resins, magnesium aluminium silica, sodium dodesyl sulphate, poloxamer, sodium glycine carbonate or mixtures thereof. Prefrably the disintegrant is sodium starch glycolate.

Suitable lubricants may comprise but not limited to magnesium stearate, calcium stearate, zinc stearate, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof. Prefrably the lubricant is magnesium stearate.

Suitable glidants may comprise but not limited to colloidal silicon dioxide (Aerosil), talc, aluminium silicate, calcium silicate, magnesium silicate, starch, magnesium carbonate, magnesium oxide or mixtures thereof. Prefrably the glidant is colloidal silicon dioxide.

Yet, according to another embodiment, the invention provides the pharmaceutical capsule composition comprising;

a. 60.0 - 80.0 % of ceftibuten dihydrate,
b. 0.1 - 2.0 % of magnesium stearate,
c. 5.0 - 15.0 % of microcrystalline cellulose,
d. 0.1 - 2.0 % of colloidal silicon dioxide,
e. 5.0 - 10.0 % of sodium starch glycolate

As a further embodiment, the invention provides a process for preparing the pharmaceutical capsule composition comprising the following steps;

a. blending ceftibuten dihydrate, microcrystalline cellulose, colloidal silicon dioxide and 2/3 of sodium starch glycolate progressively, wherein the blending time is preferably 10 min.,
b. compacting the blended mixture with a pressure of 30 - 40 bar and sieving it from 0.63 mm sieve,
c. adding the rest (1/3) of sodium starch glycolate to the powder mixture above and blending progressively for about 5 min.,
d. adding magnesium stearate to the final mixture,
e. filling the powder to the capsules.

This invention is further defined by reference to the following example. Although the examples are not intended

to limit the scope of the present invention, they should be considered in the light of the description detailed above.

**Example 1 : Ceftibuten capsule composition**

$$d_{50}= 5.5\ \mu m - 6.5\ \mu m$$

$$d_{90}= 15.0\ \mu m - 17.0\ \mu m$$

| Ingredients | Amount mg |
|---|---|
| Ceftibuten dihydrate | 435.11 |
| Magnesium stearate | 3.50 |
| Microcrystalline cellulose (pH-105) | 72.19 |
| Colloidal silicon dioxide (Aerosil) | 4.20 |
| sodium starch glycolate | 45.0 |
| **Total tablet weight** | **560.0** |

**Manufacturing Process:** Ceftibuten dihydrate, microcrystalline cellulose, colloidal silicon dioxide and 2/3 of sodium starch glycolate is blended progressively, wherein the blending time is preferably 10 min. The blended mixture then is compacting with a pressure of 35 bar and sieved with a sieve which has a pore size of 0.63 mm. The rest (1/3) of sodium starch glycolate is added to the powder mixture above and blended progressively for about 5 min. and magnesium stearate is added to the final mixture. Then the powder is filled to the capsules.

**Dissolution Profile of Ceftibuten:**

The pharmaceutical composition of this present invention (Ex.1) was tested for its dissolution profile in 1000 ml of phosphate buffer at pH 7.4, at 37ºC using a USP paddle II method rotating at 50 RPM. According to the results the ceftibuten capsule formulation of the present invention (Ex.1) has a high dissolution profile at least 85% in 15 min. as it is seen in below data.

Table 1 :

**pH 7.4 Dissolution Profile of Ceftibuten Capsule**

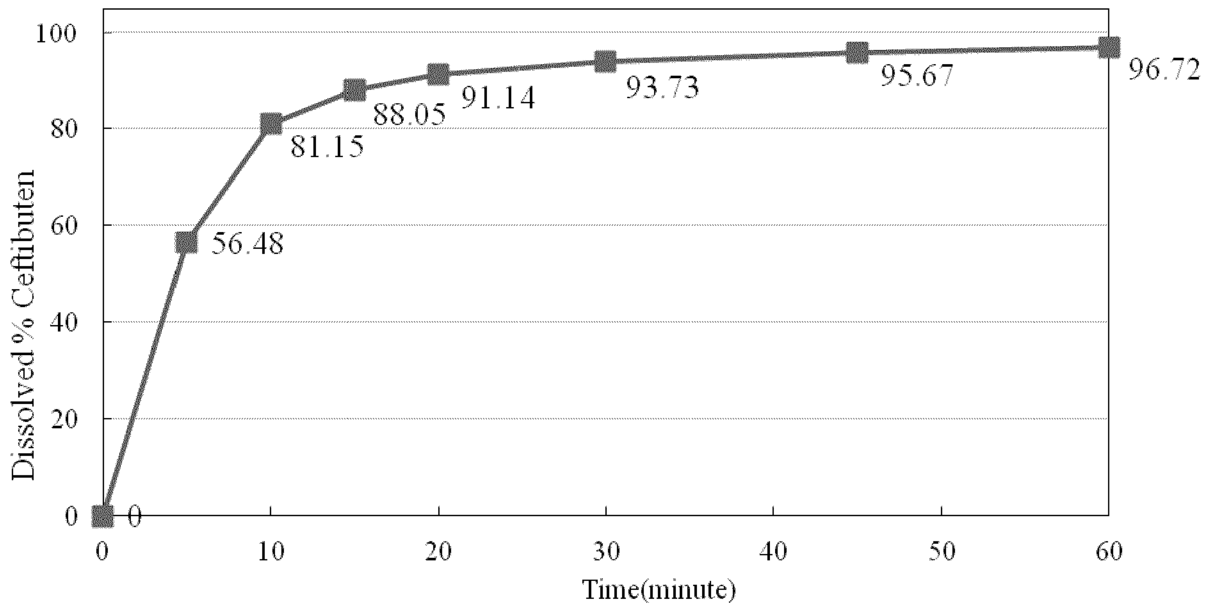


| Time (min) | % Dissolved |
|---|---|
| 0 | 0 |
| 5 | 56.48 |
| 10 | 81.15 |
| 15 | 88.05 |
| 20 | 91.14 |
| 30 | 93.73 |
| 45 | 95.67 |
| 60 | 96.72 |

## Claims

1. A pharmaceutical capsule composition comprising ceftibuten dihydrate having a particle size ($d_{90}$) between 10.0 μm and 20.0 μm and having a particle size ($d_{50}$) between 5.0 μm and 10.0 μm and at least one pharmaceutically acceptable excipient.

2. The pharmaceutical capsule composition according to claim 1, wherein ceftibuten dihydrate having a particle size ($d_{90}$) between 12.0 μm and 18.0 μm and having a particle size ($d_{50}$) between 5.0 μm and 8.0 μm.

3. The pharmaceutical composition according to claim 1 or 2, wherein ceftibuten dihydrate has a particle size ratio between $d_{50}$ and $d_{90}$ which is between 0.30 and 0.50.

4. The pharmaceutical capsule composition according to claim 1, comprising a compactor pressure between 25 bar and 45 bar.

5. The pharmaceutical capsule composition according to claim 4, wherein the compactor pressure is between 30 bar and 40 bar.

6. The pharmaceutical capsule composition according to claim 1, wherein the pharmaceutically acceptable excipient is selected from the group comprising binders, disintegrants, lubricants and glidants.

7. The pharmaceutical capsule composition according to any preceding claims, comprising;

   a. 60.0 - 80.0 % of ceftibuten dihydrate,
   b. 0.1 - 2.0 % of magnesium stearate,
   c. 5.0 - 15.0 % of microcrystalline cellulose,
   d. 0.1 - 2.0 % of colloidal silicon dioxide,
   e. 5.0 - 10.0 % of sodium starch glycolate

8. A process for preparing the pharmaceutical capsule composition according to any preceding claims, comprising the following steps;

   a. blending ceftibuten dihydrate, microcrystalline cellulose, colloidal silicon dioxide and 2/3 of sodium starch glycolate progressively, wherein the blending time is preferably 10 min.,
   b. compacting the blended mixture with a pressure of 30 - 40 bar and sieving it from 0.63 mm sieve,
   c. adding the rest (1/3) of sodium starch glycolate to the powder mixture above and blending progressively for about 5 min.,
   d. adding magnesium stearate to the final mixture,
   e. filling the powder to the capsules.

Europäisches Patentamt
European Patent Office
Office européen des brevets

# EUROPEAN SEARCH REPORT

Application Number
EP 15 19 9505

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/275552 A1 (PATEL MAHESH VITHALBHAI [IN] ET AL) 5 November 2009 (2009-11-05)<br>* page 2, paragraph 0011 *<br>* example 36 *<br>----- | 1-8 | INV.<br>A61K9/16<br>A61K31/545 |
| X<br><br>Y | DE 37 45 094 B4 (SHIONOGI & CO [JP]) 30 December 2004 (2004-12-30)<br>* page 2, paragraphs 0001, 0002, 0008 *<br>* page 8, paragraphs 0047, 0050 *<br>* examples 1-6 *<br>----- | 1-3,6,7<br><br>1-8 | |
| X<br><br>Y | CN 101 467 979 A (BEIJING HOPE HUGE PHARM SCI CO [CN]) 1 July 2009 (2009-07-01)<br>* page 5; figure *<br>* examples *<br>* claims *<br>----- | 1-3,6,7<br><br>1-8 | |

TECHNICAL FIELDS SEARCHED (IPC)

A61K

The present search report has been drawn up for all claims

1

| Place of search | Date of completion of the search | Examiner |
|---|---|---|
| The Hague | 10 March 2016 | van de Wetering, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 19 9505

This annex lists the patent family members relating to the patent documents cited in the above-mentioned European search report. The members are as contained in the European Patent Office EDP file on
The European Patent Office is in no way liable for these particulars which are merely given for the purpose of information.

10-03-2016

| Patent document cited in search report | Publication date | Patent family member(s) | Publication date |
| --- | --- | --- | --- |
| US 2009275552 A1 | 05-11-2009 | EP 2015755 A2<br>US 2009275552 A1<br>WO 2007129176 A2 | 21-01-2009<br>05-11-2009<br>15-11-2007 |
| DE 3745094 B4 | 30-12-2004 | NONE | |
| CN 101467979 A | 01-07-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4634697 A
- US 4933443 A
- US 4812561 A